# EUROPÄISCHE PATENTSCHRIFT

(11) **EP 0 656 773 B1**
(45) Veröffentlichungstag und Bekanntmachung des Hinweises auf die Patenterteilung: **02.04.1997**
(21) Anmeldenummer: 93918914.8
(22) Anmeldetag: 24.08.1993
(51) Int. Cl.: A61K 7/42, A61K 31/40, A61K 31/13, A61K 31/415, A61K 31/195

(54) **VERWENDUNG VON RADIKALFÄNGERN ALS IMMUNMODULIERENDE AGENTIEN IN KOSMETISCHEN UND DERMATOLOGISCHEN ZUBEREITUNGEN**
USE OF RADICAL CATCHERS AS IMMUNOMODULATING AGENTS IN COSMETIC AND DERMATOLOGICAL COMPOSITIONS
UTILISATION DE CAPTEURS DE RADICAUX COMME AGENTS IMMUNOMODULATEURS DANS DES COMPOSITIONS COSMETIQUES ET DERMATOLOGIQUES

(30) Priorität: 26.08.1992 DE 4228455; 25.02.1993 DE 4305788
(43) Veröffentlichungstag der Anmeldung: 14.06.1995
(73) Patentinhaber: Beiersdorf Aktiengesellschaft, D-20245 Hamburg (DE); Rijksuniversiteit te Leiden, 2312 AV Leiden (NL)
(72) Erfinder: DEGWERT, Joachim, D-21255 Tostedt (DE); GERS-BARLAG, Heinrich, D-25495 Kummerfeld (DE); VAN DEN BROEKE, Loen, T., NL-2334 EZ Leiden (NL); VAN BEIJERSBERGEN VAN HENEGOUWEN, Gerhard, M., J., NL-2396 VL Koudekerk a/d Rijn (NL)
(86) Internationale Anmeldenummer: DE9300773
(87) Internationale Veröffentlichungsnummer: WO9404128

(56) Entgegenhaltungen:
- EP-A- 0 190 685
- EP-A- 0 219 455
- EP-A- 0 238 302
- EP-A- 0 313 304
- EP-A- 0 345 362
- EP-A- 0 496 433
- EP-A- 0 500 332
- WO-A-90/10461
- WO-A-93/10755
- DE-A- 3 542 309
- FR-A- 2 233 998
- FR-A- 2 340 932
- FR-A- 2 608 425
- FR-A- 2 666 226
- US-A- 4 248 861
- IMMUNOLOGICAL REVIEWS Nr. 80 , 1984 , COPENHAGEN Seiten 87 - 102 KRIPKE 'immunological unresponsiveness induced by ultraviolet radiation'
- IMMUNOLOGYTODAY Bd. 12, Nr. 4 , 1991 Seiten 119 - 123 PAMPHILON ET AL. 'immunomodulation by ultraviolet light: clinical studies and biological effects'
- ROITT I. M . 'encyclopedia of immunology' Februar 1992 , ACADEMIC PRESS , LONDON siehe Seite 1225 - Seite 1227 "Photoimmunology " by Kripke
- INTERNATIONAL JOURNAL OF COSMETIC SCIENCE Bd. 12, Nr. 1 , Februar 1990 , LONDON Seiten 1 - 4 BADER ET AL. 'fural glucitol as free radical scavenger in the process of skin ageing'
- DATABASE WPI Week 7801, Derwent Publications Ltd., London, GB; AN 78-00904A & JP,A,52 136 925 (SANSEI SEIYAKU KK) 16. November 1977

## Beschreibung

Die vorliegende Erfindung betrifft Wirkstoffe und Zubereitungen, solche Wirkstoffe enthaltend, zur kosmetischen oder dermatologischen Behandlung und/oder Prophylaxe entzündlicher, allergischer oder autoimmunreaktiver Erscheinungen, insbesondere Dermatosen.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismen spielt.

Unter den physikalischen Umwelteinflüssen kommt dem Licht eine bedeutende Stellung zu. Die schädigende Wirkung des ultravioletten Teils der Sonnenstrahlung auf die Haut ist allgemein bekannt. Während Strahlen mit Wellenlängen kleiner als 290 nm (der sogenannte UVC-Bereich), von der Ozonschicht in der Erdatmosphäre absorbiert werden, verursachen Strahlen im Bereich zwischen 290 und 320 nm, dem sogenannten UVB-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Ultraviolettes Licht aus dem Wellenlängenbereich zwischen ca. 320 und 400 nm (UVA-Bereich) kann ebenfalls Folgeschäden der Haut hervorrufen. So ist erwiesen, daß auch UVA-Strahlung zu einer Schädigung der elastischen und kollagenen Fasern des Bindegewebes führt, was die Haut vorzeitig altern läßt (sogenanntes Photoaging), und daß sie als Ursache zahlreicher phototoxischer und photoallergischer Reaktionen zu sehen ist. Der schädigende Einfluß der UVB-Strahlung kann durch UVA-Strahlung verstärkt werden.

Bei der Entwicklung topischer Sonnenschutzmittel ist die UVB-Strahlung von besonderem Interesse, da hier das Aktionsspektrum für die akut entzündlichen Prozesse (Sonnenbrand) und chronischen Schäden (Photoaging) angesiedelt ist.

Neben diesen Effekten kann es bei UVB-Einwirkung ferner zu einer gravierenden Änderung der intraepidermalen immunologischen Situation kommen, die man als UVB-induzierte Immunsuppression bezeichnet. Dabei sind unter Umständen, je nach Strahlendosis, tiefgreifende Veränderungen der immunologischen Abläufe der Haut, mit sowohl lokalen als auch systemischen Auswirkungen, mögliche Folgen.

Immunsuppression im allgemeinen ist die Unterdrückung oder Abschwächung der Reaktivität des Immunsystems. Die UVB-induzierte Immunsuppression kann in lokale und systemische Effekte aufgegliedert werden. Letztlich umfaßt sie eine Vielzahl verschiedenster Aspekte, welche alle eine Reduktion der normalen immunologischen Abwehrmechanismen der Haut beinhalten. So wurde am Modell UVB-bestrahlter Mäuse schon sehr früh der Zusammenhang des verstärkten Tumorwachstums mit immunsuppressiver Wirkung des UVB-Lichtes in Zusammenhang gesetzt. Diese UVB-induzierte Immunsuppression wird heutzutage als Mechanismus diskutiert, mittels dessen an sich hoch immunogene, UVB-induzierte neoplastische Zellen sich der immunologischen Abwehr, und damit ihrer eigenen Zerstörung, entziehen.

Weiterhin kommt es bei UVB-Bestrahlung zu einer starken Abnahme der Kontakt-Hypersensitivitätsreaktion gegenüber manchen die Haut sensibilisierenden Agenzien. Der Grund dafür könnte in einer drastischen Verminderung der Anzahl der epidermalen Langerhanszellen liegen und/oder einer Änderung deren Morphologie und Funktionalität. Langerhanszellen aber stellen den afferenten Arm der immunologischen Abwehr der Haut dar. Ferner unterbleiben effektive Abwehrreaktionen gegen infektiöse Keime wie z.B. Candida albicans oder Herpes simples Virus.

Schließlich wird als Folge einer dermatologisch relevanten UVB-Exposition die Expression des "Intercellular Adhesion Molecule-1" auf epidermalen Keratinocyten supprimiert. Dieses zelloberflächenständige Glycoprotein (auch ICAM-1 genannt) ist eine der wichtigsten zellulären Kommunikationsstrukturen, über die direkte Zell-Zell-Kontakte zwischen epidermalen Keratinocyten und Leukocyten wie z.B. T-Lymphocyten und Monocyten reguliert werden.

Die UVB-induzierte Immunsuppression betrifft also ein breites Spektrum immunologischer Dysfunktionen, welche in einer Reduktion der normalerweise ablaufenden Abwehrreaktionen resultieren.

Es ist zwar weithin bekannt, gegen die unmittelbare Einwirkung der ultravioletten Strahlung absorbierende Agentien, nämlich die die üblichen Lichtschutzsubstanzen zu verwenden:

Zum Schutz gegen die Strahlen des UVA-Bereichs werden beispielsweise vorwiegend Derivate des Dibenzoylmethans verwendet.

Zum Schutz gegen UVB-Strahlung sind viele Verbindungen bekannt, bei denen es sich vorwiegend um Derivate des 3-Benzylidencamphers, der 4-Aminobenzoesäure, der Zimtsäure, der Salicylsäure, des Benzophenons sowie auch des 2-Phenylbenzimidazols handelt.

Es war auch weiterhin bekannt, Radikalfänger als gegen durch UV-Strahlung induzierte photoxidative Erscheinungen in der Haut wirkende Agenzien einzusetzen. Bekanntlich handelt es sich bei solchen photochemischen Reaktionsprodukten vorwiegend um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

So ist bereits vorgeschlagen worden, Vitamin E, eine Substanz mit bekannter antioxidativer Wirkung, in Lichtschutzformulierungen einzusetzen. Der Hintergrund war allerdings stets UV-Schutz durch Lichtabsorption oder Schutz gegen photooxidative Prozesse.

Aufgabe der vorliegenden Erfindung war es daher, Wirkstoffe und Zubereitungen, solche Wirkstoffe enthaltend, zur Verfügung zu stellen, mit Hilfe derer
- eine wirksame Prophylaxe gegen die UVB-Immunsuppression bewirkt werden kann
- das durch die UVB-Immunsuppression bereits geschädigte Immunsystem wieder gekräftigt werden kann.

Es war überraschend und für den Fachmann nicht vorauszusehen, daß die Verwendung eines oder mehrerer kosmetisch oder dermatologisch unbedenklicher Radikalfänger zur Herstellung kosmetischer oder dermatologischer Zubereitungen zur Behandlung und/oder Prophylaxe der durch UVB-STrahlung induzierten Immunsuppression die Lösung dieser Aufgaben darstellen würde.

Insbesondere vorteilhaft werden die Radikalfänger erfindungsgemäß gewählt aus der Gruppe der Thiole und/oder Thiolderivate, aus der Gruppe der Tocopherole und ihrer Derivate sowie aus der Gruppe bestehend aus 2,4-O-Furfurylidensorbitol und/oder dessen Alkylethern, aus der Gruppe der Carotine, aus Vitamin-A und dessen Derivaten, insbesondere Retinylpalmitat, sowie Thiodipropionsäure.

Zwar waren aus EP-A 219 455 als Bräunungsmittel wirkende Zubereitungen bekannt, welche N-Acetylcystein enthalten. Insbesondere verweist diese Schrift darauf, daß N-Acetylcystein die Pigmentbildung fördere und hauptsächlich zu diesem Zwecke eingesetzt werde. Eine gewisse zusätzliche antierythematöse Wirkung, welche am angegebenen Orte beansprucht wird, mache es für kosmetische Zubereitungen geeignet. Die vorteilhaften Eigenschaften der vorliegenden Erfindung, nämlich die vorab geschilderten Vorteile, lehrt diese Schrift indes nicht.

In EP-A 138 262 wird eine Kombination aus Panthenol, Carrageenan und Verbindungen gewählt aus der Gruppe Methionin, Cystein, N-Acetyl-Cystein, S-Acetyl-Cystein, ferner anderen Bestandteilen. Diese Kombination dient der Behandlung von durch Sonnenlicht hervorgerufenem Ekzem und Dishydrosis. Auch durch diese Schrift wird keine Lehre vermittelt, welche in die Richtung der vorliegenden Erfindung weisen könnte.

Unter Thiolen im Sinne der vorliegenden Erfindungen sind organische Verbindungen zu verstehen, welche sich durch die Gruppe -SH auszeichnen. Thiolderivate im Sinne der vorliegenden Erfindung sind organische Verbindungen, die entweder Derivate unter Beibehalt der Gruppe -SH darstellen oder aber Thioether oder Thioester, wobei dann die Gruppe -SH in die Gruppe -SR übergegangen ist.

Als mit den erfindungsgemäßen Thiolen bzw. Thiolderivaten im Sinne der vorliegenden Erfindung identisch sind ferner solche Verbindungen zu verstehen, welche durch Tautomerie, Di- oder Oligomerisierung durch Wasserstoffbrückenbindung, Hydratisierung oder andere spontane Umlagerung aus den erfindungsgemäßen Thiolen bzw. Thiolderivaten entstehen. Wenn ein Derivat durch eine andere Umlagerungsart, beispielsweise eine Alkylgruppenwanderung mit einem Isomeren im Gleichgewicht steht, so gilt dieses Isomere in die erfindungsgemäßen Thiole und Thiolderivate einbezogen.

Wenn bei den erfindungsgemäßen Thiolen mehrere mesomere oder tautomere Formen denkbar sind, wird, wie in der Chemie üblich, nur eine mesomere oder tautomere Form zur Charakterisierung angegeben.

Bevorzugte Thiole sind

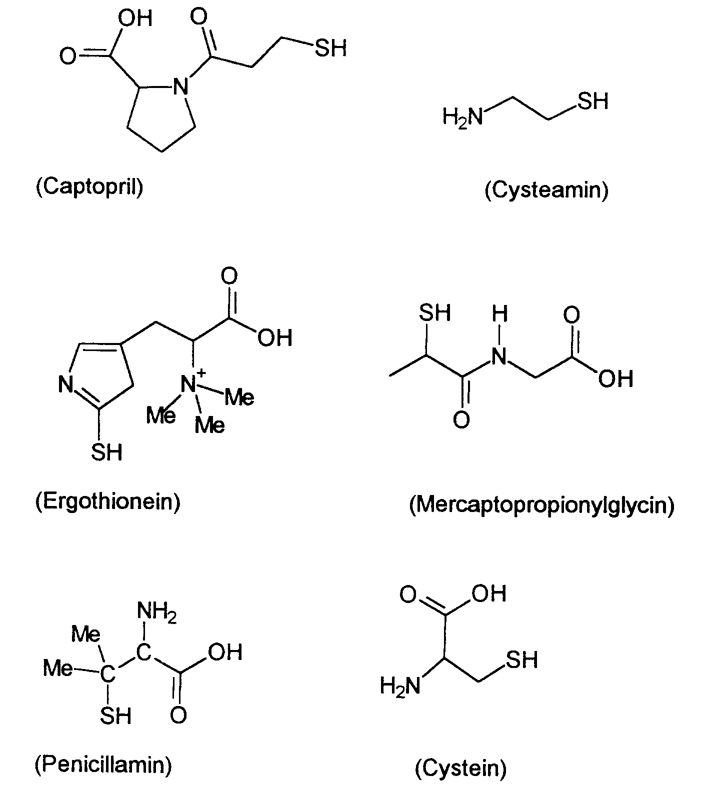

Die Wasserstoffatome an den Kohlenstoffatomen sind in dieser Beschreibung zumeist der Einfachheit halber weggelassen. Um Unklarheiten zu vermeiden, wird bei Derivaten von dieser Vereinfachung zumeist abgewichen und werden, wo es sinnvoll erscheint, Gruppierungen genauer gekennzeichnet: z.B. Me (= Methyl) oder Et (= Ethyl).

Besonders bevorzugte Derivate im erfindungsgemäßen Sinne leiten sich vom Grundkörper Cystein wie folgt ab:

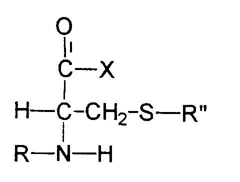

Der Rest X kann aus der Gruppe -O-R', -NRR' gewählt werden. Die Reste R, R' und R'' stellen unabhängig voneinander dar: H, C₁₋₁₈-Alkyl oder -Alkenyl, C₁₋₁₈-Acyl.

Bevorzugt ist dabei, die organischen Reste so zu wählen, daß die organischen Reste R, R' und R'' unanbhängig voneinander darstellen: H, Ethyl, Acetyl. X kann außerdem vorteilhaft die Gruppe -NH₂ symbolisieren.

Es ist aber auch gegebenenfalls vorteilhaft, die erfindungsgemäßen Thiolderivate aus der folgenden Gruppe zu wählen:

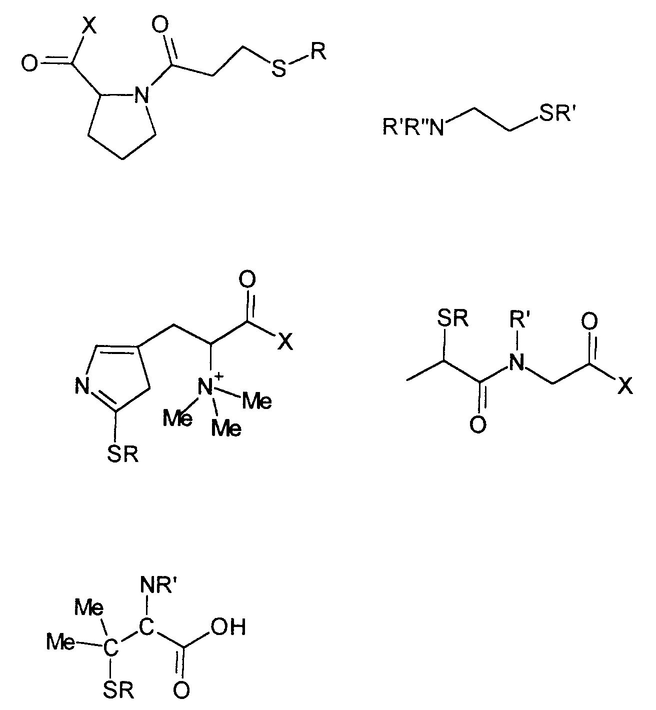

Der Rest X kann aus der Gruppe -O-R', -NRR' gewählt werden. Die Reste R, R' und R'' stellen unabhängig voneinander dar: H, C₁₋₁₈-Alkyl oder -Alkenyl, C₁₋₁₈-Acyl.

Bevorzugt ist dabei, die organischen Reste so zu wählen, daß die organischen Reste R, R' und R'' unanbhängig voneinander darstellen: H, Ethyl, Acetyl. X kann vorteilhaft die Gruppe -NH₂ symbolisieren.

Ferner sind von Vorteil die Salze bzw. Säure- oder Basenaddukte der erfindungsgemäßen Thiole bzw. Thiolderivate.

Beispiele für erfindungsgemäß besonders bevorzugte Thiole bzw. Thiolderivate sind

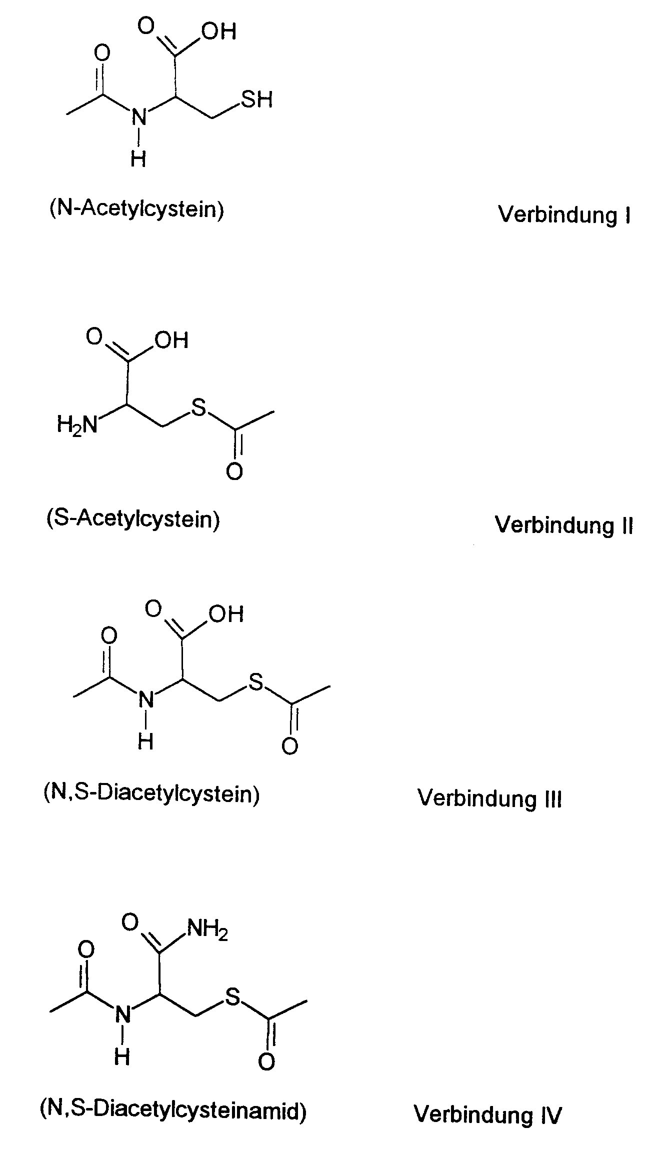

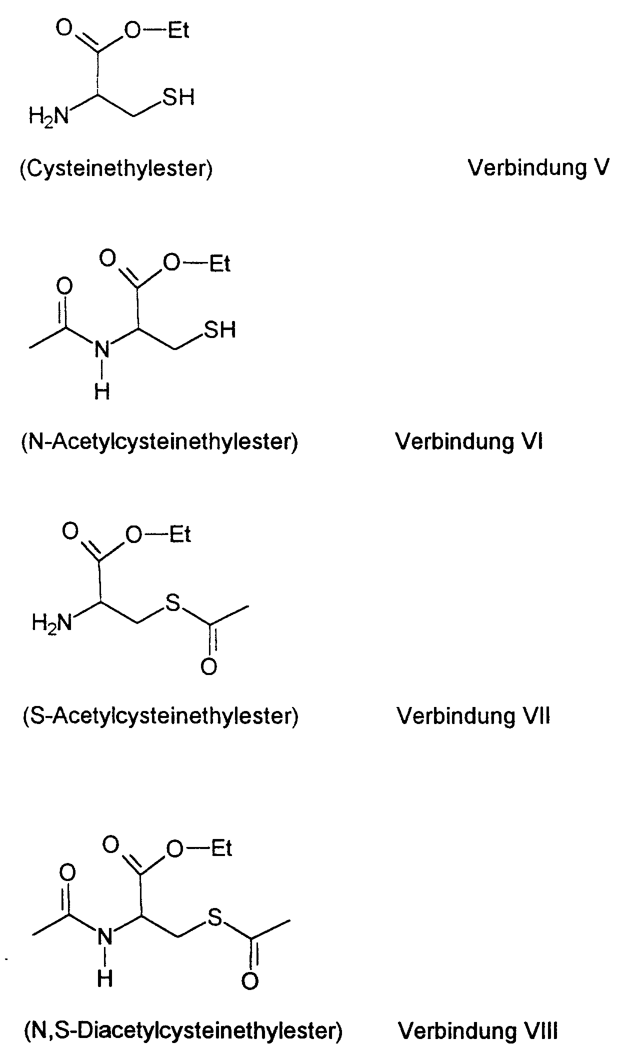

Bevorzugt sind auch die Salze bzw. Säure- oder Basenaddukte dieser besonders bevorzugten Thiole bzw. Thiolderivate.

Als erfindungsgemäß vorteilhafte Radikalfänger sind ferner 2,4-O-Furfurylidensorbitol und/oder dessen Alkylether zu verstehen.

2,4-O-Furfurylidensorbitol ist synonym mit den Bezeichnungen 2,4-O-Furfurylidenglucitol, 2,4-O-(2-Furanylmethylen)-Glucitol, 2,4-Monofurfurylidensorbitol. Es zeichnet sich durch die folgende Struktur aus: 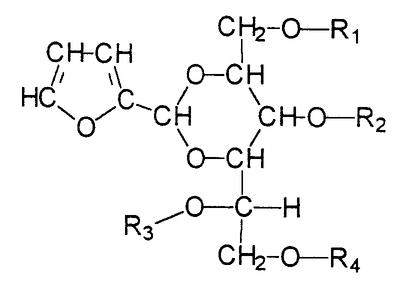 und ist in den Chemical Abstracts unter der Registraturnummer 7089-59-0 abzurufen.

R¹⁻⁴ bedeuten erfindungsgemäß unabhängig voneinander H, Methyl- und/oder Ethylgruppen. Erfindungsgemäß besonders bevorzugt sind R¹⁻⁴ identisch und stellen H oder Methylgruppen dar (2,4-Monofurfuryliden-tetra-O-methyl-sorbitol).

Die Konfiguration der vier asymmetrischen Kohlenstoffatome im vom Sorbitol abgeleiteten Molekülteil entspricht bevorzugt der der Kohlenstoffatome im natürlichen Sorbitol, obgleich auch die anderen Stereoisomere grundsätzlich vorteilhaft sind.

Die Konfiguration des asymmetrischen Kohlenstoffatoms der Furfurylidengruppe kann gleichermaßen vorteilhaft R oder S sein. Ein Racemat aus R- und S-Konfiguration, bezogen auf dieses Kohlenstoffatom, ist erfindungsgemäß von besonderem Vorteil.

Darüberhinaus können die erfindungsgemäßen Radikalfänger vorteilhaft aus der Gruppe der üblichen kosmetischen und dermatologischen Radikalfänger gewählt werden, insbesondere aus der Gruppe bestehend aus Tocopherolen und deren Derivaten, besonders α-Tocopherol bzw. α-Tocopherylestern, insbesondere α-Tocopherylacetat, ferner Sesamol, Gallensäurederivaten wie Methyl-, Ethyl-, Propyl-, Amyl-, Butyl- und Laurylgallat, dem Konyferylbenzoat des Benzoeharzes, Butylhydroxyanisol, Butylhydroxytoluol, Citronensäure, Phosphorsäure, Lecithin, Trihydroxybutyrophenon, Carotinen, Vitamin-A und dessen Derivaten, insbesondere Retinylpalmitat, Ascorbinsäure, Ascorbylpalmitat, Dilaurylthiodipropionat, Distearylthiodipropionat, Monoisopropylcitrat, Phytinsäure und deren Salze, Thiodipropionsäure und EDTA sowie EDTA-Derivaten.

Die Tocopherole, auch Vitamin E genannt, leiten sich vom Grundkörper Tocol ((2-Methyl-2-(4,8,12-trimethyltridecyl)chroman-6-ol) ab und sind durch folgende Strukturen gekennzeichnet:

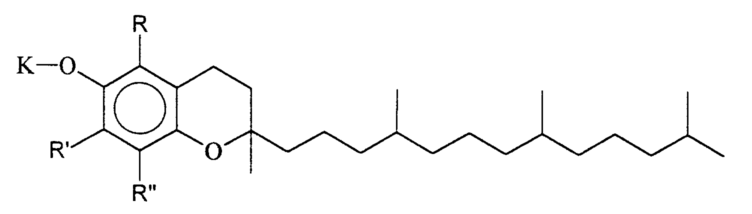

Dabei stellt K entweder H oder einen Acylrest dar, R, R' und R'' bedeuten unabhängig voneinander H oder eine Methylgruppe, z.B.:
- R = R' = R'' = K = H: : Tocol
- R = R' = R'' = Methyl, K = H: : α-Tocopherol
- R = R'' = Methyl, R' = K = H: : β-Tocopherol
- R = R' = R'' = Methyl, K = -C(O)-CH₃: : α-Tocopherolacetat
und weitere Varianten. In diesen Estern gilt für den Acylrest K:

K = -C(O)-R'''',

wobei R'''' einen Alkyl oder Alkenylrest mit 1 bis 21 Atomen darstellen kann. Besonders bevorzugt ist R'''' = Methyl.

Dem natürlich am häufigsten vorkommenden und bedeutendsten α-Tocopherol kommt die Konfiguration 2R,4'R,8'R zu. Es wird gelegentlich auch RRR-α-Tocopherol genannt.

Die erfindungsgemäß bevorzugten Tocopherolderivate sind das α-Tocopherol und seine Ester, insbesondere das α-Tocopherylacetat.

Es ist aus der EP-Patentanmeldungsschrift 345 362 bekannt, 2,4-Furfurylidensorbitol in kosmetischen und dermatologischen Formulierungen einzusetzen in solchen Formulierungen dient es in erster Linie als abfangendes Agens für freie Radikale, welche unter anderem durch UV-Strahlung hervorgerufen werden können.

Die erfindungsgemäße Verwendung des 2,4-Furfurylidensorbitols zur Herstellung kosmetischer oder dermatologischer Zubereitungen zur Prophylaxe der durch UVB-Strahlung induzierten Immunsuppression wurde indes nicht durch den Stand der Technik nahegelegt.

Ferner ist aus US-PS 4,144,325 und 4,248,861 sowie aus zahlreichen anderen Dokumenten bekannt, Vitamin E in kosmetischen und dermatologischen Lichtschutzformulierungen einzusetzen.

Die erfindungsgemäße Verwendung des Vitamins E und seiner Derivate zur Herstellung kosmetischer oder dermatologischer Zubereitungen zur Prophylaxe der durch UVB-Strahlung induzierten Immunsuppression wurde indes nicht durch den Stand der Technik nahegelegt.

Die erfindungsgemäßen kosmetischen und/oder dermatologischen Formulierungen können wie üblich zusammengesetzt sein und zur Prophylaxe und/oder zur Behandlung der Haut im Sinne einer dermatologischen Behandlung oder einer Prophylaxe und/oder Behandlung im Sinne der Kosmetik dienen. Sie können aber auch in Schminkprodukten in der dekorativen Kosmetik eingesetzt werden. Sie enthalten bevorzugt 0,01 Gew.-% bis 10 Gew.-%, insbesondere aber 0,1 Gew.-% bis 6 Gew.-%, bezogen auf das Gesamtgewicht eines oder mehrerer Radikalfänger.

Es ist erfindungsgemäß insbesondere vorteilhaft, Kombinationen aus mehreren Radikalfängern zu verwenden, insbesondere, wenn wenigstens eine der Komponenten gewählt wird aus der Gruppe der Thiole bzw. Thiolderivate und 2,4-O-Furfurylidensorbitol und dessen Alkylethern.

Vorteilhaft ist insbesondere, Kombinationen aus 2,4-O-Furfurylidensorbitol bzw. dessen Alkylethern und einem oder mehreren Tocopherolen bzw. dessen Derivaten zu verwenden.

Zur Anwendung werden die erfindungsgemäßen Radikalfänger bzw. Zubereitungen, solche Radikalfänger enthaltend, vorzugsweise ein oder mehrere Thiole bzw. Thiolderivate bzw. ein oder mehrere Tocopherole bzw. deren Derivate bzw. 2,4-O-Furfurylidensorbitol bzw. dessen Alkylether in der für Kosmetika oder Dermatika üblichen Weise auf die Haut in ausreichender Menge aufgebracht.

Besonders bevorzugt sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfönsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Radikalfängern verwendet werden können, soll selbstverständlich nicht limitierend sein.

Gegenstand der Erfindung ist auch die Kombination eines oder mehrerer erfindungsgemäßer Radikalfänger mit einem oder mehreren UVB-Filtern bzw. erfindungsgemäße kosmetische oder dermatologische Zubereitungen, welches auch einen oder mehrere UVB-Filter enthalten.

Es kann auch von Vorteil sein, einen oder mehrere erfindungsgemäße Radikalfänger mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner werden vorteilhafte Zubereitungen erhalten, wenn die erfindungsgemäßen Radikalfänger mit UVA- und UVB-Filtern kombiniert werden.

Kosmetische Zubereitungen, erfindungsgemäße Radikalfänger enthaltend, können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und/oder UVB-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen Zubereitungen zum Schutz der Kopfhaut vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, mindestens ein erfindungsgemäßer Radikalfänger im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie mindestens einen erfindungsgemäßen Radikalfänger. Die Menge der verwendeten Polymeren liegt z.B. zwischen 0,1 und 10 Gew.-%, bevorzugt zwischen 0,1 und 3 Gew.-%.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

### Beispiel 1

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung I | 0,300 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 2

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung II | 0,500 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 3

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung III | 2,500 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 4

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung IV | 3,000 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 5

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Verbindung V | 1,750 |
| ^{Na}3HEDTA | 1,500 |
| Parfum, Konservierungsmittel, Farbstoffe | nach Belieben |
| H₂O VES | ad 100,000 |

### Beispiel 6

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,750 |
| Verbindung VI | 2,500 |
| ^{Na}3HEDTA | 1,500 |
| Parfum, Konservierungsmittel, Farbstoffe | nach Belieben |
| H₂O VES | ad 100,000 |

### Beispiel 7

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 1,000 |
| Verbindung VII | 0,700 |
| ^{Na}3HEDTA | 1,500 |
| Parfum, Konservierungsmittel, Farbstoffe | nach Belieben |
| H₂O VES | ad 100,000 |

### Beispiel 8

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Verbindung VIII | 1,600 |
| ^{Na}3HEDTA | 1,500 |
| Parfum, Konservierungsmittel, Farbstoffe | nach Belieben |
| H₂O VES | ad 100,000 |

Die nachfolgenden Angaben zu den Zusammensetzungen der Wirkstoffkombinationen I - V beziehen sich auf das Gesamtgewicht der Wirkstoffkombinationen.

### Wirkstoffkombination I

| | Gew.-% |
|---|---|
| Furfurylidensorbitol | 10,00 |
| Vitamin E-Acetat | 90,00 |

### Wirkstoffkombination II

| | Gew.-% |
|---|---|
| Furfurylidensorbitol | 25,00 |
| Vitamin E-Acetat | 75,00 |

### Wirkstoffkombination III

| | Gew.-% |
|---|---|
| Furfurylidensorbitol | 50,00 |
| Vitamin E-Acetat | 50,00 |

### Wirkstoffkombination IV

| | Gew.-% |
|---|---|
| Furfurylidensorbitol | 75,00 |
| Vitamin E-Acetat | 25,00 |

### Wirkstoffkombination V

| | Gew.-% |
|---|---|
| Furfurylidensorbitol | 90,00 |
| Vitamin E-Acetat | 10,00 |

### Beispiel 9

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination I | 0,300 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 10

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination II | 0,500 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 11

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination III | 2,500 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 12

| | Gew.-% |
|---|---|
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination IV | 3,000 |
| ZnSO₄ | 0,700 |
| Na₄EDTA | 0,300 |
| Parfum, Konservierungsmittel, Farbstoffe | nach Belieben |
| H₂O VES | ad 100,000 |

### Beispiel 13

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Wirkstoffkombination V | 1,750 |
| ^{Na}3HEDTA | 1,500 |
| Parfum, Konservierungsmittel, Farbstoffe | nach Belieben |
| H₂O VES | ad 100,000 |

### Beispiel 14

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,750 |
| Wirkstoffkombination I | 2,500 |
| ^{Na}3HEDTA | 1,500 |
| Parfum, Konservierungsmittel, Farbstoffe | nach Belieben |
| H₂O VES | ad 100,000 |

### Beispiel 15

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 1,000 |
| Wirkstoffkombination II | 0,700 |
| ^{Na}3HEDTA | 1,500 |
| Parfum, Konservierungsmittel, Farbstoffe | nach Belieben |
| H₂O VES | ad 100,000 |

### Beispiel 16

| | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Wirkstoffkombination III | 1,600 |
| ^{Na}3HEDTA | 1,500 |
| Parfum, Konservierungsmittel, Farbstoffe | nach Belieben |
| H₂O VES | ad 100,000 |

### Wirkungsnachweis:

Nachfolgend sollen anhand eines Versuches die vorteilhaften Eigenschaften der vorliegenden Erfindung verdeutlicht werden.

Als Modell für die immunsupprimierende Wirkung der UVB-Strahlung wurde die UVB-Mixed-Lymphocyte-Reaction-Methode (UVB-MLR) verwendet. Die UVB-MLR ist eine Methode, um Effekte von Prüfsubstanzen auf die UVB-induzierte Suppression einer zellulären Immunantwort zu analysieren. Es handelt sich dabei um eine Modifikation der MLR, eines immunologischen in-vitro-Standardverfahrens, welches als Maß für die Aktivierung und Funktionalisierung des T-Lymphocytensystems dient.

Die Prüfsubstanzen werden hierzu den Zellkulturen in verschiedenen Konzentrationen zugefügt. Als Bestrahlungsquelle diente eine Phillips TL 20W/12-Lampe.

7,5 mJ UVB/cm², 15 mJ UVB/cm², und 30 mJ UVB/cm² wurden als Bestrahlungsdosis eingesetzt.

Mononukleäre Zellen des peripheren Blutes zweier gesunder humaner Spender werden mittels einer Dichte-Gradienten-Zentrifugation aufgereinigt und gemeinsam in Mikrotiter-Platten kultiviert. Die Einzelkultur setzt sich hierbei zusammen aus 3,0 * 10⁵ mit Mitocytin behandelten Stimulatorzellen (Spender A) und 2,5 * 10⁵ Responderzellen (Spender B) (Inkubation bei 37° C, 7,5 % CO₂, 10 % FCS (Fötales Kälberserum) in RPMI 1640 Medium).

In einer "one way"-MLR sind die Stimulatorzellen durch die Behandlung mit Mitocytin physiologisch arretiert, so daß lediglich sie als zelluläres Antigen für die Responderzellen dienen, deren Proliferation über den Einbau von ³H-Thymidin bestimmt wird. Die Responderzellen werden von den Stimulatorzellen nicht mehr als Antigen erkannt.

Der Einbau von ³H-Thymidin wird nach Separation aller Zellen, also Stimulator- und Responderzellen analysiert. Die Menge des inkorporierten ³H-Thymidins korreliert mit der Fähigkeit zur Immunantwort: je weniger ³H-Thymidin inkorporiert wird, desto stärker ist die UVB-Immunsuppression.

Um nun den Einfluß von UV-Licht auf die Zellproliferation (Responderzellen) zu bestimmen, werden die Stimulatorzellen vor ihrer Inkubation mit den Responderzellen mit der entsprechenden UVB-Dosis bestrahlt. In entsprechenden Parallelansätzen ist die entsprechende Prüfsubstanz während der Bestrahlung im Kulturmedium zugegen.

Durch Vergleiche der in Ab- und Anwesenheit von Prüfsubstanzen erreichbaren Proliferation der Responderzellen, nach erfolgter Ko-Kultivierung mit den Stimulatorzellen, ermöglicht Aussagen über Immunprotektive Eigenschaften dieser Substanzen auf der Ebene UVB-induzierter Immunsuppression.

### Im Versuch getestete erfindungsgemäße, gegen UVB-Immunsuppression wirksame Agentien:

Verbindungen I - VIII (Thiole bzw. Thiolderivate), 2,4-O-Furfurylidensorbitol, α-Tocopherol α-Tocopherylacetat, Citronensäure, Retinylpalmitat, Ascorbinsäure, Ascorbylpalmitat wurden als Prüfsubstanz verwendet.

### Ergebnis:

Es konnte bei allen vorab genannten getesteten erfindungsgemäßen, gegen UVB-Immunsuppression wirksamen Agentien eine signifikante immunprotektive Wirkung beobachtet werden.

### Literatur zur verwendeten Methode:

Blain, B. et al. (1964), **Blood** 23, S.108,
Meo, T. et al. (1975), **Transplant. Proc.** 7, S.127
Mommaas, A. M. et al. (1990), **J.Invest.Dermatol.** 95, S.313
Marinus, C. G. et al. (1991), **J.Invest.Dermatol.** 97, S.629

## Patentansprüche

1. Verwendung eines oder mehrerer kosmetisch oder dermatologisch unbedenklicher Radikalfänger zur Herstellung kosmetischer oder dermatologischer Zubereitungen zur Behandlung und/oder Prophylaxe der durch UVB-Strahlung induzierten Immunsuppression.

2. Verwendung nach Anspruch 1, dadurch gekennzeichnet, daß der oder die Radikalfänger gewählt werden aus der Gruppe der Thiole und/oder Thiolderivate, aus der Gruppe der Tocopherole und ihrer Derivate, aus der Gruppe bestehend aus 2,4-O-Furfurylidensorbitol und/oder dessen Alkylethern, aus der Gruppe der Carotine, aus der Gruppe aus Vitamin-A und dessen Derivaten, insbesondere Retinylpalmitat, sowie Thiodipropionsäure.

3. Verwendung nach Anspruch 2, dadurch gekennzeichnet, daß das oder die Thiole bzw. Thiolderivate gewählt werden aus der Gruppe der Verbindung, die durch die generische Formel 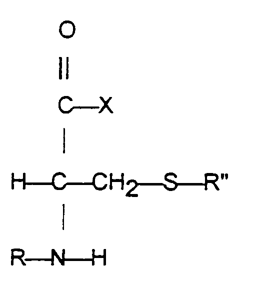 beschrieben wird, wobei der Rest X aus der Gruppe -O-R', -NRR' gewählt wird und die Reste R, R' und R'' unabhängig voneinander darstellen: H, C₁₋₁₈ -Alkyl oder -Alkenyl, C₁₋₁₈ -Acyl.

4. Verwendung nach Anspruch 3, dadurch gekennzeichnet, daß die organischen Reste so gewählt werden, daß der Rest X aus der Gruppe -O-R', -NRR' gewählt wird, und daß R, R' und R'' unanbhängig voneinander darstellen: H, Ethyl, Acetyl.

## Claims

1. Use of one or more cosmetically or dermatologically acceptable free radical scavengers for the preparation of cosmetic or dermatological preparations for the treatment and/or prophylaxis of the immunosuppression induced by UVB radiation.

2. Use according to Claim 1, characterized in that the free radical scavenger(s) is(are) selected from the thiols and/or thiol derivatives group, from the tocopherols group and their derivatives, from the group consisting of 2,4-O-furfurylidenesorbitol and/or its alkyl ethers, from the group of carotenes, from the group of vitamin A and its derivatives, in particular retinyl palmitate, and also thiodipropionic acid.

3. Use according to Claim 2, characterized in that the thiol(s) or thiol derivative(s) is(are) selected from the compound group which is described by the generic formula 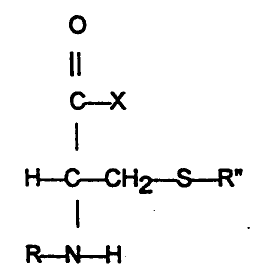 the radical X being selected from the group -O-R' and -NRR' and the R, R' and R'' radicals independently of one another being: H, C₁₋₁₈-alkyl or -alkenyl, or C₁₋₁₈-acyl.

4. Use according to Claim 3, characterized in that the organic radicals are selected such that the radical X is selected from the group -O-R' and -NRR', and in that R, R' and R'' independently of one another represent: H, ethyl or acetyl.

## Revendications

1. Utilisation d'un ou plusieurs capteurs de radicaux, inoffensifs du point de vue cosmétique ou dermatologique, pour la préparation de compositions cosmétiques ou dermatologiques destinées au traitement et ou à la prophylaxie de l'immunosuppression induite par un rayonnement UVB.

2. Utilisation selon la revendication 1, caractérisée en ce que le ou les capteurs de radicaux sont choisis parmi les thiols et/ou dérivés de thiols, les tocophérols et leurs dérivés, le 2,4,O-furfurylidènesorbitol et/ou ses éthers alkyliques, les carotènes, la vitamine A et ses dérivés, en particulier le palmitate de rétinyle, ainsi que l'acide thiodipropionique.

3. Utilisation selon la revendication 2, caractérisée en ce que le ou les thiols ou dérivés de thiols sont choisis parmi les composés de formule générique 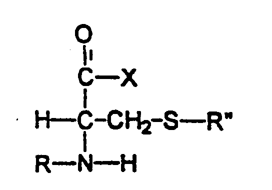 dans laquelle le radical X est choisi parmi -O-R', -NRR', et les radicaux R, R' et R'' représentent, indépendamment les uns des autres, un atome d'hydrogène ou un groupe alkyle ou alcényle en C₁₋₁₈, ou un groupe acyle en C₁₋₁₈.

4. Utilisation selon la revendication 3, caractérisée en ce que les radicaux organiques sont choisis de manière que le radical X soit choisi parmi -O-R', -NRR', et R, R' et R'' représentent, indépendamment les uns des autres, un atome d'hydrogène ou le groupe éthyle ou acétyle.
